(19) **Europäisches Patentamt / European Patent Office / Office européen des brevets**

(11) **EP 4 455 744 B1**

(12) **FASCICULE DE BREVET EUROPEEN**

(45) Date de publication et mention de la délivrance du brevet:
**24.12.2025 Bulletin 2025/52**

(21) Numéro de dépôt: **24172755.1**

(22) Date de dépôt: **26.04.2024**

(51) Classification Internationale des Brevets (IPC):
*G02B 5/22* (2006.01)    *G01J 3/42* (2006.01)
*G01N 21/3504* (2014.01)    *G02B 5/28* (2006.01)
*G01N 21/03* (2006.01)    *G01N 21/45* (2006.01)
*G01N 21/3518* (2014.01)

(52) Classification Coopérative des Brevets (CPC):
**G02B 5/22; G01J 3/26; G01J 3/42; G01N 21/3504; G02B 5/281; G02B 5/284;** G01N 21/253; G01N 21/3581; G01N 2021/177; G01N 2021/3522

(54) **ELÉMENT INTERFÉROMÉTRIQUE, DISPOSITIF DE DÉTECTION D'UN COMPOSÉ COMPRENANT UN ÉLÉMENT INTERFÉROMÉTRIQUE ET MÉTHODE DE DÉTECTION D'UN COMPOSÉ**

INTERFEROMETRISCHES ELEMENT, VORRICHTUNG ZUR DETEKTION EINER VERBINDUNG MIT EINEM INTERFEROMETRISCHEN ELEMENT UND VERFAHREN ZUR DETEKTION EINER VERBINDUNG

INTERFEROMETRIC ELEMENT, DEVICE FOR DETECTING A COMPOUND COMPRISING AN INTERFEROMETRIC ELEMENT AND METHOD FOR DETECTING A COMPOUND

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **28.04.2023 FR 2304354**

(43) Date de publication de la demande:
**30.10.2024 Bulletin 2024/44**

(73) Titulaire: **Commissariat à l'Energie Atomique et aux Energies Alternatives**
**75015 Paris (FR)**

(72) Inventeur: **DUSSOPT, Laurent**
**75015 Paris (FR)**

(74) Mandataire: **Atout PI Laplace**
**Immeuble Up On**
**25 Boulevard Romain Rolland**
**CS 40072**
**75685 Paris Cedex 14 (FR)**

(56) Documents cités:
**US-A- 4 417 815**    **US-A1- 2003 112 443**
**US-A1- 2007 057 187**    **US-A1- 2021 018 431**

• **KUULIALA L ET AL: "Spoilage evaluation of raw Atlantic salmon (Salmo salar) stored under modified atmospheres by multivariate statistics and augmented ordinal regression", INTERNATIONAL JOURNAL OF FOOD MICROBIOLOGY, vol. 303, 20 May 2019 (2019-05-20), pages 46 - 57, XP085705415, ISSN: 0168-1605, DOI: 10.1016/ J.IJFOODMICRO.2019.04.011**

## Description

### Domaine technique :

La présente invention concerne le domaine de la détection de particules et plus particulièrement de la détection de particules par interférométrie optique.

### Technique antérieure :

Dans de nombreux domaines d'application comme l'agro-alimentaire, la défense ou la chimie, la détection et l'identification de particules sont nécessaires dans le but de donner l'alerte d'une éventuelle attaque ou contamination. De nombreuses techniques connues de l'homme de l'art permettent de déterminer une composition chimique d'un échantillon.

Par exemple, des senseurs chimiques et des biosenseurs permettent d'obtenir une détection rapide et un contrôle en temps réel de l'interaction entre l'échantillon ou les composés à détecter et le senseur. De tels senseurs utilisent une couche de détection chimique ou biomoléculaire afin de reconnaître un composé à détecter en se liant à ce dernier. Cette couche peut par exemple comprendre des molécules comme des anticorps, des enzymes, des hormones, de l'ADN, des récepteurs neurotransmetteurs, etc...

Ce type de senseurs n'est pas réutilisable car l'étape de liage entre le composé à détecter et la couche de détection n'est en général pas réversible. Ces capteurs sont donc utilisés une fois puis jetés.

Les capteurs en optique intégré fournissent une alternative attrayante à ces senseurs chimiques. En effet, les techniques de fabrication de guides d'onde intégré sur des puces optiques par photolithographie et microfabrication permettent une production en masse, à bas coût et répétable de capteur en optique intégré. La plupart de ces capteurs en optique intégré sont des interféromètres de type Mach-Zender (ou MZI pour Mach-Zender interferometer en anglais).

La spectroscopie infrarouge à transformée de Fourier (FTIR) est une technique analytique extrêmement répandue dans laquelle les molécules de l'échantillon absorbent le rayonnement incident modifiant ainsi leurs énergies de vibration. Selon les liaisons et les fonctions chimiques présentes dans l'échantillon, un spectre infrarouge (IR) caractéristique est obtenu.

Outre la spectroscopie FTIR, il existe une multitude de techniques permettant d'identifier des particules au sein d'un échantillon comme la spectrométrie Raman, la spectrométrie sur plasma induite par laser (LIBS), ou encore la méthode de photo-fragmentation induite par la fluorescence (PF-LIF), spécifique aux liaisons NO2.

Les techniques mettant en jeu l'absorption infrarouge peuvent être déclinées suivant plusieurs modalités.

Une première méthode est l'imagerie IR « passive » en transmission ou en rétrodiffusion, par absorption directe de la lumière par la particule. Dans ce cas, la puissance optique collectée diminue en présence de l'échantillon. Il est par exemple connu d'analyser un gaz généré par la décomposition de l'échantillon soumis à un laser intense pulsé en rétrodiffusion moyen infrarouge (MIR). Le rayonnement détecté est le rayonnement thermique (de type corps noir) des objets constituant la scène observée. Celle-ci est observée par un imageur infrarouge dont la bande spectrale de détection est soit large bande (couvre l'ensemble sur spectre IR), soit restreint à une partie du spectre. Un traitement d'image utilise les contrastes, à la fois dans le domaine spectral et spatial, entre les deux types d'images et en déduit un niveau d'absorption et donc une concentration en gaz. Ce type de système est bien adapté à des fortes concentrations en gaz et des scènes vastes, par exemple pour surveiller les émissions d'un site industriel. Cette technique d'imagerie permet de détecter et d'identifier des nuages de gaz. Il n'est pas possible d'utiliser cette technique pour des objets de petites dimensions comme des particules puisque le chemin optique parcouru dans l'objet est trop petit.

Une deuxième méthode consiste à effectuer de l'imagerie thermique « active ». En effet, il existe des capteurs actifs intégrant dans un module une source infrarouge, des filtres et des détecteurs permettant de détecter une variation de signal en fonction de la présence du gaz recherché. Ce type de système peut accéder à un très haut niveau de sensibilité. En revanche, il peut être encombrant, onéreux et nécessite une source d'énergie. Ces inconvénients peuvent être rédhibitoires si on souhaite détecter un gaz dans un environnement confiné, peu accessible, ou demandant des technologies bas coût.

Enfin, une dernière technique est la photo-acoustique qui peut être séparée en 4 étapes :

(1) absorption du rayonnement laser par le gaz excitant ainsi les niveaux d'énergies rotationnels, électroniques et vibrationnels;
(2) dans le cas d'excitations ro-vibrationnelles, désexcitation du gaz préférentiellement par des collisions moléculaires qui vont se traduire par un transfert d'énergie de rotation/vibration et énergie cinétique créant un chauffage localisé du gaz ;
(3) génération d'une onde acoustique et d'une onde thermique provoquée par l'expansion due au chauffage du gaz ;
(4) détection par le microphone du signal acoustique. L'amplitude de vibration du microphone est représentative de la concentration du gaz et la longueur d'onde du rayonnement laser absorbée par le gaz indique sa composition.

Cette dernière méthode est intéressante mais elle ne permet pas d'avoir une image d'une zone. Il faudrait scanner le laser sur l'échantillon ce qui demande de l'instrumentation et du temps de mesure. De plus, cette technique requiert une source laser modulable en intensité ou en longueur d'onde, ce qui implique un cout important du dispositif de détection.

Un document de l'art antérieur est connu sous la forme de US 2021/018431.

L'invention vise à pallier certains problèmes de l'art antérieur en fournissant un élément interférométrique passif permettant la détection à distance d'un composé prédéterminé, possiblement à travers une paroi matérielle transparente à la gamme de longueur d'onde utilisée. L'élément interférométrique de l'invention est particulièrement intéressant pour des applications bas coût (par exemple le contrôle de produits de grande consommation), ou dans des applications industrielles en atmosphère hostile (fortes ou basses températures, atmosphères explosives ou corrosives).

**Résumé de l'invention** :

A cet effet, un objet de l'invention est un élément interférométrique destiné à un dispositif de détection d'au moins un composé présentant une absorption résonante sur une région spectrale prédéterminée centrée sur une longueur d'onde de résonance $\lambda_r$, ledit élément interférométrique comprenant au moins un sous-ensemble de détection optimisé pour ladite longueur d'onde de résonance $\lambda_r$ et comprenant :

- au moins deux cavités optiques de type Fabry-Pérot présentant une résonance à ladite longueur d'onde de résonance $\lambda_r$, chaque cavité comprenant une couche réfléchissante (CR) à ladite longueur d'onde de résonance $\lambda_r$ et une couche partiellement transparente à ladite longueur d'onde de résonance $\lambda_r$,

- une couche d'encapsulation imperméable au(x) composé(s) à détecter et encapsulant au moins une cavité optique, dite cavité référence, de manière à ce que ladite cavité référence soit dépourvue dudit composé à détecter entre la couche réfléchissante et la couche partiellement transparente, la couche d'encapsulation n'encapsulant pas la seconde cavité optique, dite cavité sensible, et la couche partiellement transparente de la cavités sensible étant perméable au(x) composé(s) à détecter de manière à ce que la cavité sensible puisse comprendre le ou les composé(s) à détecter entre la couche réfléchissante et la couche partiellement transparente.

De manière préférentielle, l'élément interférométrique comprend une pluralité de cavités sensibles et une pluralité de cavités références, et les cavités sensibles et les cavités références sont agencées selon une disposition prédéterminée de manière à pouvoir déterminer une position des cavités sensibles et des cavités références par traitement d'une image dudit élément interférométrique. De manière encore préférentielle, ladite disposition prédéterminée est telle que les cavités sensibles et les cavités références sont disposées de manière alternées selon une ligne ou une pluralité de lignes préférentiellement parallèles.

Selon un mode de réalisation, l'élément interférométrique comprend une mire optique adaptée de manière à pouvoir déterminer une orientation et une position dudit élément interférométrique par traitement d'une image dudit élément interférométrique.

Selon un mode de réalisation, la ou les cavités sensibles sont adaptées pour présenter un coefficient de réflexion $R_s(\lambda_r)$ à la longueur d'onde de résonance $\lambda_r$ et la ou les cavités références sont adaptées pour présenter un coefficient de réflexion $R_f(\lambda_r)$ à la longueur d'onde de résonance $\lambda_r$ tels que $R_f(\lambda_r) - R_s(\lambda_r) > 1\%$, et préférentiellement $R_f(\lambda_r) - R_s(\lambda_r) > 2\%$, pour une concentration de 1% du ou des composé(s) à détecter entre la couche réfléchissante et la couche partiellement transparente des cavités sensibles.

Selon un mode de réalisation, l'élément interférométrique comprend une pluralité de sous ensemble de détections chacun optimisé pour une longueur d'onde de résonance respective et différente de l'autre ou des autres longueurs d'onde de résonance. De manière préférentielle, les longueurs d'onde de résonance sont distantes les unes des autres de moins de 50%. Alternativement, les longueurs d'onde de résonance sont distantes les unes des autres d'au moins 5%. Selon une variante de ce mode de réalisation, les cavités optiques de chaque sous-ensemble de détection comprennent, entre ladite couche partiellement transparente et ladite couche réfléchissante, une couche diélectrique identique respectivement associée audit sous ensemble de détection, un indice de réfraction et une épaisseur de ladite couche diélectrique respectivement associée audit sous ensemble de détection étant différente d'un indice de réfraction et d'une épaisseur de la ou des couches diélectriques respectivement associée(s) au(x) autres(s) sous ensemble(s) de détection et étant adaptés de manière à ce que les cavités optiques présentent une même épaisseur.

Selon un mode de réalisation, la couche partiellement transparente est séparée d'une distance p $\times$ $\lambda_r/2$, de la couche réfléchissante avec $p \in \mathbb{N}^* > 2$, préférentiellement p > 4.

Un autre objet de l'invention est un dispositif de détection d'au moins un composé présentant une absorption résonante sur une région spectrale centrée sur une longueur d'onde de résonance $\lambda_r$, ledit dispositif comprenant :

- une source de lumière adaptée pour générer un premier faisceau incident présentant au moins ladite longueur d'onde de résonance $\lambda_r$

- un élément interférométrique selon l'invention

- agencé de manière à ce que le premier faisceau illumine la couche partiellement transparente de l'au moins une cavité sensible et la couche partiellement transparente de l'au moins une cavité de référence

- un capteur comprenant une pluralité de pixels et adapté pour acquérir une image du premier faisceau incident réfléchi par l'élément interférométrique, dite première image,

- une unité de traitement reliée au capteur et configurée pour détecter une éventuelle présence du ou des composé(s) à détecter à partir d'une comparaison d'une intensité entre, d'une part, au moins une première région de pixels où est détecté le premier faisceau incident réfléchi par ladite au moins une cavité sensible et d'autre part au moins une deuxième région de pixels où est détecté le premier faisceau incident réfléchi par ladite au moins une cavité de référence.

Un autre objet de l'invention est un dispositif de détection d'au moins un composé présentant une absorption résonante sur une région spectrale centrée sur une longueur d'onde de résonance $\lambda_r$, ledit dispositif comprenant

- une source de lumière adaptée pour générer un premier faisceau incident présentant au moins ladite longueur d'onde de résonance $\lambda_r$ et pour générer un deuxième faisceau incident ne présentant aucune longueur d'onde comprise dans ladite absorption résonante et présentant au moins une longueur d'onde dite longueur d'onde hors résonance $\lambda_{nr}$,

- un élément interférométrique selon l'invention agencé de manière à ce que le premier et le deuxième faisceau illuminent la couche partiellement transparente d'au moins une cavité sensible et la couche transparente d'au moins une cavité de référence

- un capteur comprenant une pluralité de pixels et adapté pour acquérir une image du premier faisceau incident réfléchi par l'élément interférométrique, dite première image, et une image du deuxième faisceau incident réfléchi par l'élément interférométrique , dite deuxième image

- une unité de traitement reliée au capteur et configurée pour détecter une éventuelle présence du ou des composé(s) à détecter à partir d'une comparaison d'une intensité de la première image et de la deuxième image.

Un autre objet de l'invention est une méthode de détection d'au moins un composé présentant une absorption résonante sur une région spectrale centrée sur une longueur d'onde de résonance $\lambda_r$, ladite méthode

comprenant les étapes suivantes :

- générer un premier faisceau incident présentant au moins ladite longueur d'onde de résonance $\lambda_r$ et un deuxième faisceau incident ne présentant aucune longueur d'onde comprise dans ladite absorption résonante et présentant au moins une longueur d'onde dite longueur d'onde hors résonance $\lambda_{nr}$

- illuminer un élément interférométrique selon l'invention avec le premier et le deuxième faisceau de manière à ce qu'ils illuminent la couche partiellement transparente d'au moins une cavité sensible et la couche transparente d'au moins une cavité de référence

- acquérir une image du premier faisceau incident réfléchi par l'élément interférométrique, dite première image, et une image du deuxième faisceau incident réfléchi par l'élément interférométrique, dite deuxième image

- détecter une éventuelle présence du ou des composé(s) à détecter à partir d'une comparaison d'une intensité de la première image et de la deuxième image.

Selon un mode de réalisation, ladite détection comprend les étapes suivantes :

A- calculer une troisième image par la différence entre la deuxième image et la première image

B- dans la troisième image, comparer l'intensité entre, d'une part, au moins une première région de pixels où est détecté le premier faisceau incident réfléchi par ladite au moins une cavité sensible, et d'autre part, au moins une deuxième région de pixels où est détecté le premier faisceau incident réfléchi par ladite au moins une cavité de référence.

De manière préférentielle, la détection comprend une étape A0, mise en œuvre avant ladite étape A, consistant à recaler la première image et la deuxième image.

Selon un mode de réalisation, ladite détection est effectuée lorsqu'une moyenne $Imoy_1$ de l'intensité de ladite au moins une première région et une moyenne $Imoy_2$ de l'intensité de ladite au moins une deuxième région sont telles que $\frac{|Imoy_1 - Imoy_2|}{Imoy_1} > S$, avec $S$ compris entre 0.5% et 5%.

Selon un mode de réalisation, la première image et la deuxième image sont acquises simultanément ou dans un intervalle de temps inférieur à 5 secondes, préférentiellement inférieur à 1 seconde.

**Brève description des figures** :

D'autres caractéristiques, détails et avantages de l'invention ressortiront à la lecture de la description faite en référence aux dessins annexés donnés à titre d'exemple et qui représentent, respectivement :

[Fig.1], une vue schématique d'un dispositif 1 de détection d'au moins un composé selon l'invention,

[Fig.2A], une vue en coupe selon un plan yz d'un élément interférométrique selon un mode de réalisation de l'invention,

[Fig.2B], l'évolution du coefficient de réflexion des cavités sensibles (courbe C1) et des cavités références (courbe C2) en fonction de la longueur d'onde,

[Fig.2C], l'évolution du coefficient de réflexion des cavités optiques en fonction de la concentration en composé dans le milieu ambiant de l'élément interférométrique,

[Fig.3A], un exemple des images acquises pour un élément interférométrique selon un mode de réalisation de l'invention,

[Fig.3B], un élément interférométrique selon un mode de réalisation de l'invention

[Fig.4], une représentation graphique de l'évolution du coefficient de réflexion d'une cavité selon un mode de réalisation en fonction de la longueur d'onde, pour cinq valeurs différentes de concentration en $H_2S$ dans la cavité,

[Fig.5A], [Fig.5B], un exemple d'une image hors résonance et à la résonance respectivement, et des histogrammes des pixels de ces deux images acquises pour un élément interférométrique selon un mode de réalisation de l'invention.

[Fig.6], l'évolution de la valeur du coefficient de réflexion en fonction de la tangente de pertes à la résonance, pour trois distances L différentes.

[Fig.7], un mode de réalisation dans lequel l'élément interférométrique comprend deux sous-ensembles de détection chacun optimisé pour une longueur d'onde de résonance respective et différente de l'autre,

[Fig.8], un objet de l'invention qui est un emballage par exemple alimentaire comprenant un aliment AL et comprenant l'élément interférométrique de l'invention.

Dans les figures, sauf contre-indication, les éléments ne sont pas à l'échelle et les références identiques désignent des éléments identiques.

**Description détaillée :**

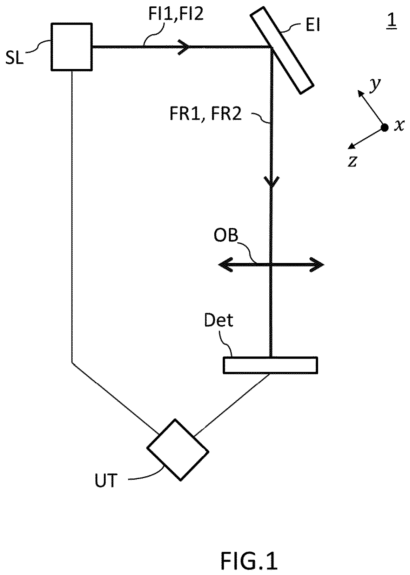

La figure 1 illustre de manière simplifiée un dispositif 1 selon l'invention de détection d'au moins un composé C. Le dispositif 1 comprend essentiellement une source de lumière SL, un élément interférométrique EI selon l'invention, un capteur Det et une unité de traitement UT.

Pour simplifier la description, on suppose que le dispositif 1 est optimisé pour détecter un unique composé C sauf quand l'inverse sera explicitement précisé. Le composé à détecter C est connu et prédéterminé et possède une absorption résonante sur une région spectrale centrée sur une longueur d'onde de résonance $\lambda_r$. A titre d'exemple non limitatif, le composé à détecter est le sulfure d'hydrogène ($H_2S$). Le spectre d'absorption du gaz $H_2S$ a plusieurs bandes de forte absorption dans le domaine térahertz, en en particulier une raie centrée à 612 GHz. Des travaux ont montré l'apparition de ce gaz et l'augmentation de sa concentration à l'intérieur des emballages de produits alimentaires en quelques jours, en faisant un indicateur de la dégradation du produit (voir L. Kuuliala et al., "Spoilage evaluation of raw Atlantic salmon (Salmo salar) stored under modified atmospheres by multivariate statistics and augmented ordinal regression", International Journal of Food Microbiology, vol. 303, p. 46-57, août 2019, doi: 10.1016/j.ijfoodmicro.2019.04.011.).

La source de lumière SL est adaptée pour générer *a minima* un premier faisceau incident FI1 présentant au moins la longueur d'onde de résonance $\lambda_r$. La source de lumière SL n'est pas spécifique à l'invention et est par exemple une source laser.

Selon un mode de réalisation principal illustré dans la figure 1, la source de lumière SL est en outre adaptée pour générer un deuxième faisceau incident FI2 ne présentant aucune longueur d'onde comprise dans l'absorption résonante du composé C et présentant au moins une longueur d'onde dite longueur d'onde hors résonance $\lambda_{nr}$. Par « un deuxième faisceau incident FI2 ne présentant aucune longueur d'onde comprise dans l'absorption résonante du composé C », on entend ici que le deuxième faisceau ne présente aucune longueur d'onde comprise dans un intervalle spectral dépendant de la largeur spectrale de l'absorption résonante, qui est par exemple tel que $[0.98 \times \lambda_r; 1.02 \times \lambda_r]$, et préférentiellement aucune longueur d'onde dans un intervalle spectral tel que $[0.96 \times \lambda_r; 1.04 \times \lambda_r]$.

Selon une première alternative du mode de réalisation principal, la source de lumière SL est une source adaptée pour générer simultanément le premier et le deuxième faisceau. Par exemple, la source de lumière SL comprend une source laser monochromatique émettant à la longueur d'onde de résonance $\lambda_r$ et

comprend une architecture optique permettant de générer au moins la longueur d'onde hors résonance $\lambda_{nr}$ à partir de la longueur d'onde de résonance $\lambda_r$, typiquement en prélevant une portion du premier faisceau FI1 et en le doublant en fréquence. Alternativement, la source laser SL comprend deux sources laser respectivement monochromatique à la longueur d'onde hors résonance $\lambda_{nr}$ et à la longueur d'onde de résonance $\lambda_r$. Alternativement, la source SL est une source large bande couvrant une bande spectrale suffisamment large pour englober la longueur d'onde hors résonance $\lambda_{nr}$ et la longueur d'onde de résonance $\lambda_r$.

Selon une deuxième alternative, la source de lumière SL est apte à générer le premier et le deuxième faisceau successivement dans le temps. Par exemple la source SL comprend une source laser accordable en longueur d'onde ou deux sources laser fonctionnant en alternance et respectivement monochromatique à la longueur d'onde hors résonance $\lambda_{nr}$ et à la longueur d'onde de résonance $\lambda_r$.

Alternativement, selon un mode de réalisation secondaire, la source de lumière SL est adaptée pour générer uniquement le premier faisceau incident FI1. Dans la suite, on décrira d'abord le fonctionnement du dispositif selon le mode de réalisation principal puis le fonctionnement du dispositif selon le mode de réalisation secondaire.

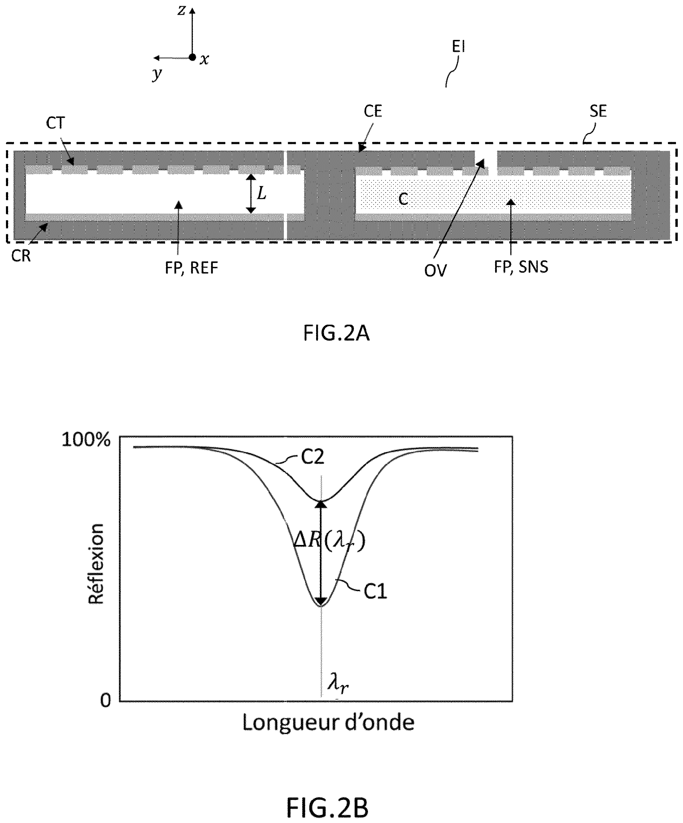

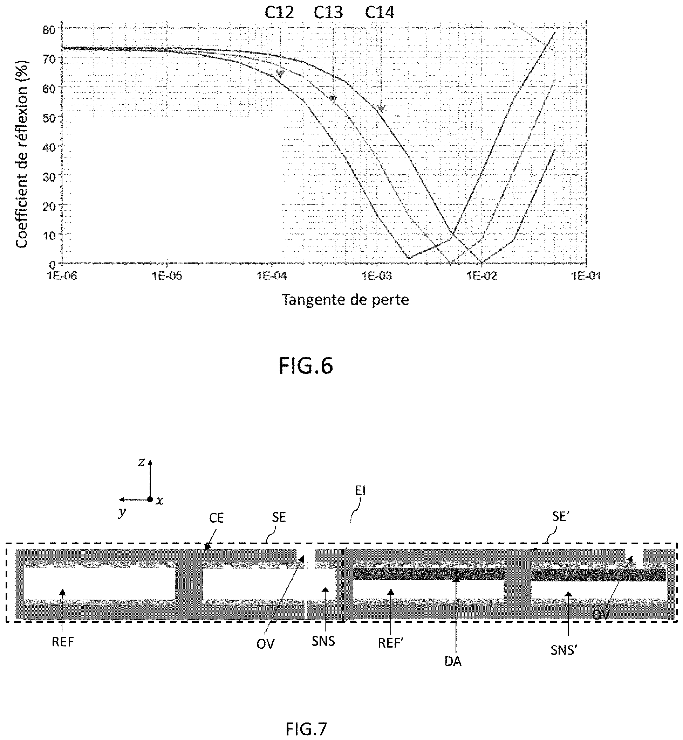

L'élément interférométrique **EI** comprend au moins un sous ensemble de détection SE (non visible en figure 1 mais visible en figure 2A) optimisé pour la longueur d'onde de résonance $\lambda_r$ de manière à permettre la détection du composé C. Selon certains modes de réalisation, l'élément interférométrique EI comprend plusieurs sous ensemble de détection chacun optimisé pour détecter un ou plusieurs composés respectifs (voir figure 7 décrite plus loin). Il est important de souligner que l'élément interférométrique **EI** de l'invention est un élément passif ne nécessitant aucune alimentation électrique afin de permettre la détection du composé C.

La figure 2A est une vue en coupe selon un plan yz d'un élément interférométrique **EI** selon un mode de réalisation de l'invention.

Le sous ensemble SE optimisé pour permettre la détection du composé C comprend une pluralité de cavités optiques FP de type Fabry-Pérot présentant résonance à la longueur d'onde de résonance $\lambda_r$. Il est en outre nécessaire que les cavités optiques FP ne présentent pas une absorption résonante à la longueur d'onde hors résonance $\lambda_{nr}$. Pour cela, on choisira par exemple $\lambda_{nr}$ tel que, pour chaque bande de résonance d'indice $i$ des cavités FP, centrée sur une longueur d'onde $\lambda_{r,i}$ et de largeur à mi-hauteur $\delta\lambda_i$, on a

$$\lambda_{nr} \notin \left[\lambda_{r,i} - \frac{\delta\lambda_i}{2}; \lambda_{r,i} + \frac{\delta\lambda_i}{2}\right].$$

A titre d'exemple non limitatif, le sous ensemble SE de l'élément interférométrique EI de la figure 2A comprend deux cavités FP alignées selon la direction $y$.

Chacune des cavités FP comprend une couche réfléchissante CR à la longueur d'onde de résonance $\lambda_r$ et une couche partiellement transparente CT à la longueur d'onde de résonance $\lambda_r$. Typiquement, chaque couche réfléchissante CR présente un coefficient de réflexion supérieur à 99%, préférentiellement supérieur à 99,5% à la longueur d'onde de résonance $\lambda_r$ et chaque couche partiellement transparente CT présente un coefficient de réflexion compris entre 90% et 99%, préférentiellement compris entre à 97% et 99% à la longueur d'onde de résonance $\lambda_r$.

Par exemple, les couches CT et CR sont des couches métalliques, la couche CT pouvant comprendre une structuration spatiale afin d'augmenter sa transmission.

Alternativement, la couche CT et/ou la couche CR est/sont un miroir de Bragg constitué d'un empilement de matériaux diélectriques d'indices optiques différents et d'épaisseurs multiples du quart de la longueur d'onde de résonance.

Les couches CT et CR de chaque cavité sont empilées selon une direction d'empilement (z dans l'illustration de la figure 2A) de manière à être séparée par une distance $L$. Cette distance est adaptée en fonction du ou des milieux séparant les couches CT et CR pour que chaque cavité FP présente une résonance à la longueur d'onde de résonance $\lambda_r$. Comme cela sera précisé plus en détail dans la figure 6, plus cette distance L est importante plus la sensibilité du capteur sera importante.

De manière connue, un interféromètre de Fabry-Pérot présente une différence de marche entre chaque rayon transmis par l'interféromètre qui vaut $2n.L.\cos(\theta)$, avec $\theta$ un angle de propagation des rayons entre les couches CR et CR de l'interféromètre et avec un milieu d'indice n séparant les couches CT et CR. On peut démontrer que la transmission d'un interféromètre de Fabry-Pérot est maximale lorsque cette différence de marche est égale à un multiple de la longueur d'onde des rayons se propageant dans l'interféromètre. En incidence normale, la transmission de chaque cavité FP est donc maximale pour une **distance** $L = \frac{p \times \lambda_r}{2n}$, avec $p \in \mathbb{N}^*$. Aussi, en supposant un milieu d'indice $n$ séparant les couches CT et CR des cavités FP de l'élément interférométrique EI de l'invention, la distance L de séparation des couches CT et CR est telle que

$$L = \frac{p \times \lambda_r}{2n}, \text{ avec } p \in \mathbb{N}^*.$$

Le sous-ensemble SE de l'élément interférométrique EI comprend en outre une couche d'encapsulation CE imperméable au composé à détecter C et transparente aux longueurs d'onde $\lambda_r$ et $\lambda_{nr}$. Par « transparente », on entend ici que la couche d'encapsulation CE présente une transmission supérieure à 80%, préférentiellement supérieure à 90% pour les longueurs d'onde $\lambda_r$ et $\lambda_{nr}$.

Cette couche d'encapsulation CE est disposée de manière à encapsuler un premier sous ensemble de cavité(s) optique(s) la ou les cavités références REF-de manière à ce que chaque cavité référence soit dépourvu du composé à détecter entre les couches CT et CR. Ainsi, selon un mode de réalisation, les couches CT et CR de la ou des cavités références sont séparées par du vide ou par un milieu neutre (typiquement un gaz non résonant aux longueurs d'onde $\lambda_r$ et $\lambda_{nr}$ par exemple de l'azote).

De plus, la couche d'encapsulation CE n'encapsule pas un second sous ensemble de cavité(s) optique(s) : la ou les cavités sensibles SNS. Ainsi, la ou les cavités sensibles SNS de l'élément interférométrique peuvent comprendre le composé à détecter C entre leur couche réfléchissante CR et leur couche partiellement transparente CT lorsque le milieu ambiant comprend le composé C. Par exemple, comme illustré dans la figure 2A, la couche d'encapsulation CE comprend une ouverture OV de manière ce que la cavité sensible SNS soit exposée au milieu ambiant. Pour cela, il est nécessaire que la couche partiellement transparente CT de chaque cavité sensible SNS soit perméable au composé à détecter C.

Ainsi, la ou les cavités sensibles et la ou les cavités références présentent un coefficient de réflexion identique (ou quasiment identique) très élevé (typiquement supérieur à 99%) à longueur d'onde hors résonance $\lambda_{nr}$ et présentent un coefficient de réflexion inférieur et différent à longueur d'onde de résonance $\lambda_r$. Ce résultat est illustré dans la figure 2B qui représente l'évolution du coefficient de réflexion des cavités sensibles (courbe C1) et des cavités références (courbe C2) en fonction de la longueur d'onde.

Sur la courbe C2, on observe que les cavités références présentent un coefficient de réflexion proche de 100% sur la majeure partie du spectre et légèrement inférieure à la longueur d'onde de résonance $\lambda_r$ en raison des pertes plus élevées générées par le phénomène de résonance électromagnétique.

Sur la courbe C1, on observe que les cavités sensibles présentent également un coefficient de réflexion proche de 100% sur la majeure partie du spectre car le composé C n'a pas d'effet significatif sur la transmission en l'absence de résonance. En revanche, le coefficient de réflexion est nettement réduit par rapport à la cavité référence à la longueur d'onde de résonance $\lambda_r$ compte tenu de l'absorption du composé C.

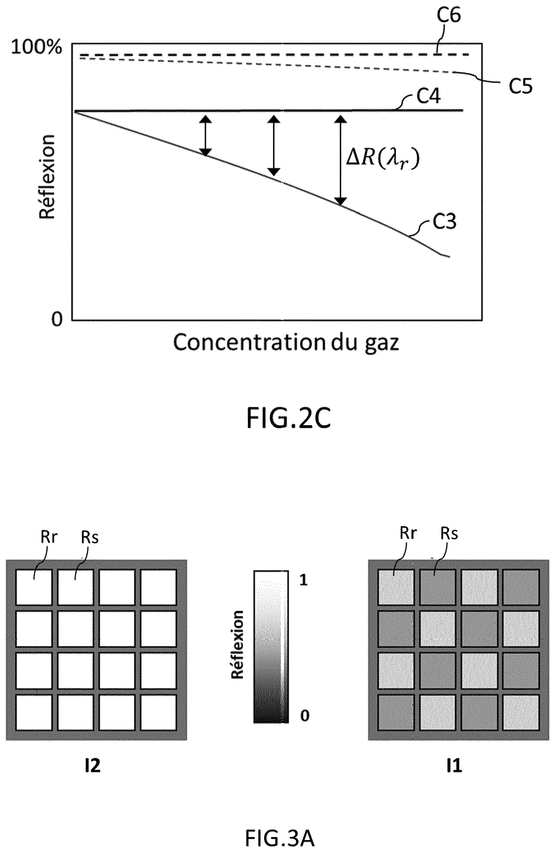

On note $\Delta R(\lambda_r) = R_r(\lambda_r) - R_s(\lambda_r)$ la différence entre la valeur $R_s(\lambda_r)$ du coefficient de réflexion à la longueur d'onde de résonance des cavités références $\lambda_r$ et la valeur $R_r(\lambda_r)$ du coefficient de réflexion à la longueur d'onde de résonance des cavités sensibles. On peut démontrer que cette différence $\Delta R(\lambda_r)$ est sensiblement proportionnelle à la concentration du composé C. Ce résultat est observable dans la figure 2C qui illustre l'évolution du coefficient de réflexion des cavités optiques en fonction de la concentration en composé C dans le milieu ambiant de l'élément interférométrique **EI** (et donc entre les couches CR et CT des cavités sensibles).

Les courbes C3 et C5 de la figure 2C illustrent respectivement l'évolution du coefficient de réflexion des cavités sensibles en fonction de la concentration en composé C à la longueur d'onde de résonance et à la longueur d'onde hors résonance.

Les courbes C4 et C6 de la figure 2C illustrent l'évolution du coefficient de réflexion des cavités références en fonction de la concentration en composé C à la longueur d'onde de résonance et à la longueur d'onde hors résonance respectivement.

Les cavités références présentent un coefficient de réflexion constant ou quasi-constant. La valeur de ce coefficient de réflexion est proche de 100% et est sensiblement plus élevé pour la longueur d'onde hors résonance.

Les cavités sensibles présentent un coefficient de réflexion élevé et légèrement décroissant avec la concentration de gaz en dehors de la résonance (C5) et une réflexion fortement décroissante à la résonance (C3).

De manière préférentielle, les cavités sensibles et références sont adaptées - par le biais des coefficients de réflexion des couches CR et CT- pour présenter un coefficient de réflexion $R_s(\lambda_r)$ et coefficient de réflexion $R_r(\lambda_r)$ respectivement tels que $\Delta R(\lambda_r) = R_r(\lambda_r) - R_s(\lambda_r) > 1\%$, et préférentiellement $\Delta R(\lambda_r) > 2\%$, pour une concentration de 1% du composé à détecter entre la couche réfléchissante et la couche partiellement transparente des cavités sensibles. Cette valeur permet de faciliter la détection du composé C.

Comme illustré en figure 1, l'élément interférométrique EI est agencé de manière à être illuminé par le premier et le deuxième faisceau FI1, FI2. Plus précisément, les faisceaux FI1, FI2 illuminent la couche CT d'au moins une cavité sensible SNS et la couche CT d'au moins une cavité de référence REF.

Le capteur Det comprend une pluralité de pixels et est adapté pour acquérir une image du premier faisceau incident réfléchi FR1 par l'élément interférométrique, dite première image I1 ou image à résonance I1. De plus, le capteur est apte à acquérir une image du deuxième faisceau incident réfléchi FR2 par l'élément interférométrique, dite deuxième image I2 ou image hors résonance I2.

Le capteur Det n'est pas spécifique à l'invention et sera adapté par l'homme de l'art en fonction de la source de lumière SL sans sortir du cadre de l'invention.

Par exemple, selon le mode de réalisation dans lequel la source de lumière SL est une source large bande, le capteur Det comprend une matrice de pixels associés à un système de filtres spectral aptes à capturer des images à $\lambda_r$ et à $\lambda_{nr}$. Typiquement, le système de filtres spectral est une roue à filtres spectraux disposée optiquement en amont de la matrice de pixels. Alternativement, le capteur Det est une matrice de pixels multispectrale et comprend par exemple plusieurs sous-ensembles de matrices de pixels ayant chacun un filtre spectral distinct.

Selon un autre mode de réalisation dans lequel la source émet une pluralité de faisceaux monochromatiques successivement ou simultanément, le capteur Det est par exemple une matrice de pixels large bande comme une caméra CCD ou CMOS.

Enfin, l'unité de traitement UT est reliée au capteur Det et est configurée pour détecter une éventuelle présence du composé à détecter C à partir d'une comparaison d'une intensité de la première image I1 et de la deuxième image I2.

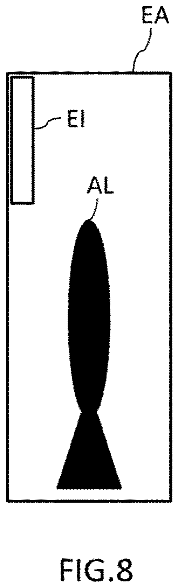

Ainsi, l'élément interférométrique EI passif de l'invention permet la détection à distance du composé C. Selon la longueur d'onde de résonance $\lambda_r$ utilisée, cette détection peut être effectuée au travers d'un élément optiquement transparent, par exemple un emballage de type alimentaire (voir figure 8 décrite plus loin). L'élément interférométrique de l'invention est particulièrement intéressant pour des applications bas coût (par exemple le contrôle de produits de grande consommation), ou dans des applications industrielles en atmosphère hostile (fortes ou basses températures, atmosphères explosives ou corrosives).

En effet, comme évoqué plus haut, la structure de l'élément interférométrique implique que les régions de pixels où sont détectés la réflexion des faisceaux FR1, FR2 par les cavités sensibles, dites premières régions Rs, présentent une intensité différente entre la première et la deuxième image I1, I2. A l'inverse, les régions de pixels où sont détectés la réflexion des faisceaux FR1, FR2 par les cavités références, dites deuxièmes régions Rr, présentent une intensité égale ou sensiblement égale entre la première et la deuxième image I1, I2. Par une comparaison de l'intensité de ces régions Rs et Rr, l'unité de traitement UT permet une détection du composé C au sein des cavités sensibles.

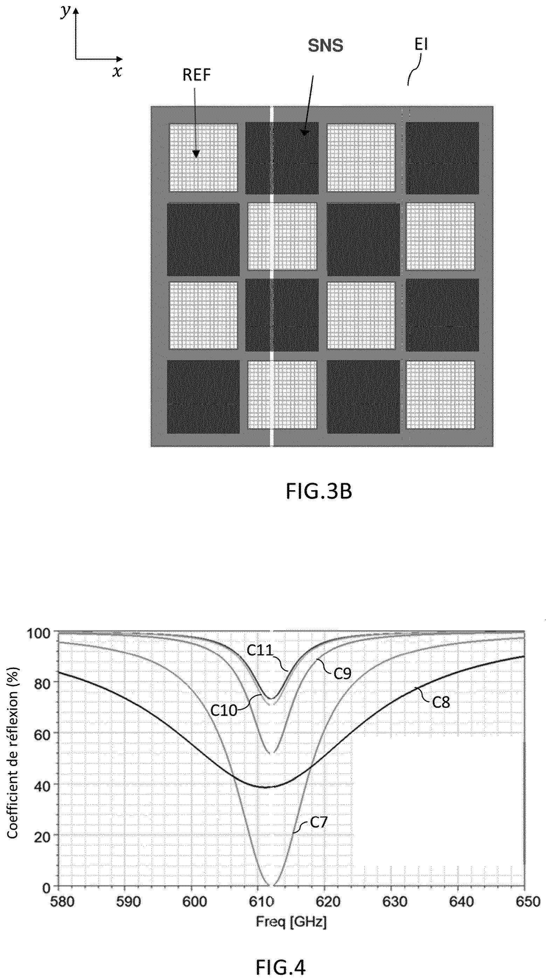

Ce résultat est illustré dans les figures 3A et 3B. Plus précisément, la figure 3A est un exemple des images I1 et I2 acquises pour un élément interférométrique EI en présence du composé à détecter C dans lequel les cavités sensibles SNS et références REF sont agencées selon l'illustration de la figure 3B.

Dans le mode de réalisation de la figure 3B, à titre d'exemple non limitatif, les cavités sensibles SNS et références REF sont disposées en « damier », c'est-à-dire que les cavités sont disposées de manière alternées en une pluralité de lignes parallèles. Cette disposition est avantageuse car elle permet des mesures d'intensité dans les images des cavités sensibles et références proches et donc associées à des chemins optiques quasi-identiques.

Sur la figure 3A, on observe que les régions Rs et Rr associées respectivement aux cavités sensibles et références présentent une intensité quasi-identique dans l'image hors résonance I2 et présentent une intensité différente dans l'image à résonance I1. En effet, compte tenu de la présence du composé C entre les couches CT et CR des cavités sensibles SNS qui absorbent le faisceau FI1 mais pas le faisceau FI2, le coefficient de réflexion des cavités sensibles SNS est inférieur à celui des cavités références REF pour le faisceau FI1 (voir figure 2B).

C'est pourquoi, comme illustré dans l'image I1 de la figure 3A, l'intensité des régions Rs est inférieure à celle des régions Rr.

Selon un mode de réalisation du mode de réalisation principal, l'unité de traitement est adaptée pour que l'étape de détection comprenne une première étape consistant à calculer une troisième image par la différence entre la deuxième image I2 et la première image I1.

Ainsi, à condition que les images I1 et I2 soient acquises simultanément ou dans un intervalle de temps suffisamment proche, la première étape permet de s'affranchir des caractéristiques du chemin optique source de lumière SL-élément interférométrique EI-capteur Det (pertes de propagation, pertes optiques, orientation du capteur, phénomènes de réflexion/diffraction dans l'environnement du système, etc.). Le calcul de la troisième image permet aussi de localiser les régions Rs et Rr plus facilement que dans les images I1 et I2.

Par « un intervalle de temps suffisamment proche », on entend ici que la première image et la deuxième image sont acquises dans un intervalle de temps inférieur à 5 secondes, préférentiellement inférieur à 1 seconde.

Dans une deuxième étape, l'unité de traitement UT est configurée pour comparer l'intensité entre au moins une première région Rs et au moins une deuxième région de pixels R2 dans la troisième image. Lorsque l'unité de traitement identifie une différence d'intensité notable entre la ou les régions Rs et la ou les régions Rr, l'unité de traitement UT considère que le composé C est détecté.

De manière préférentielle, l'unité de traitement UT considère que le composé C est détecté lorsqu'une moyenne $Imoy_1$ de l'intensité des premières régions Rs et une moyenne $Imoy_2$ de l'intensité des deuxième régions Rr sont telles que :

$$\Delta I = \frac{|Imoy_1 - Imoy_2|}{Imoy_1} > S$$

avec $S$ un seuil prédéterminé dépendant de :

- la concentration minimale en composé C entre les couches CT et CR que l'on souhaite détecter,

- le coefficient de réflexion des cavités sensibles à $\lambda_r$ et $\lambda_{nr}$

- le coefficient de réflexion des cavités références à $\lambda_r$ et $\lambda_{nr}$

De manière connue en soi, les coefficients de réflexion sont déterminés par les coefficients des couches CT et CR.

Typiquement, ce seuil S est compris entre 0.5% et 5% pour une concentration minimale de 1%.

De manière préférentielle, la première étape de calcul de la troisième image est préférentiellement précédée d'une étape préliminaire de recalage entre la première et la deuxième image. Cette étape de recalage est connue en soi et peut être mise en œuvre par toute méthode connue de l'homme de l'art. En traitement d'image, le recalage est une technique qui consiste en la « mise en correspondance d'images », dans le but de les comparer ou les combiner.

Selon un mode de réalisation, cette étape de recalage est effectuée par des procédés de traitement d'image basée sur la corrélation 2D des images I1 et I2.

Selon un mode de réalisation, compatible avec tous les modes de réalisation de l'invention, les cavités sensibles et les cavités références sont agencées selon une disposition prédéterminée de manière à pouvoir déterminer une position des cavités sensibles et des cavités références par traitement d'image. Ainsi, l'étape de recalage est mise en œuvre par l'unité de traitement par traitement d'image, grâce à cette disposition prédéterminée préalablement stockée dans l'unité de traitement UT.

Selon un autre mode de réalisation, compatible avec tous les modes de réalisation de l'invention, l'élément interférométrique comprend une mire optique. Cette mire optique est adaptée de manière à pouvoir déterminer une orientation et une position de l'élément interférométrique à partir d'une image. La mire optique facilite ainsi le recalage des images I1 et I2.

De manière préférentielle, l'élément interférométrique EI comprend une pluralité de cavités sensibles et de cavités références (par exemple plus de cinq cavités sensibles et plus de cinq cavités références). Cela permet d'effectuer une moyenne des régions Rs et Rr dans les I1 et I2 (ou directement dans la troisième image) et ainsi d'effectuer l'étape de comparaison d'intensité des régions Rs et Rr mise en œuvre par l'unité de traitement UT sur la moyenne des régions Rs et la moyenne des régions Rr. Ainsi, on peut s'affranchir des dispersions venant des détecteurs, des cavités résonantes ou de l'environnement (par exemple un objet occultant une partie de l'élément interférométrique).

Selon un mode de réalisation, l'unité de traitement UT est en outre configurée pour déterminer la concentration en composé C, à partir de la valeur de $\Delta I$. En effet, comme évoqué précédemment, la différence $\Delta R(\lambda_r)$ entre la valeur du coefficient de réflexion entre les cavités sensibles SNS et celle des cavités références REF, à la longueur d'onde de résonance, est sensiblement proportionnelle à la concentration du composé C. Naturellement, cette différence $\Delta R(\lambda_r)$ est proportionnelle à la valeur de $\Delta I$. Il est donc possible d'effectuer une calibration par des mesures préalables avec des concentrations prédéterminées en composé C et ainsi associer une valeur de $\Delta I$ à une concentration en composé C.

Comme évoqué précédemment, dans le mode de réalisation secondaire, la source SL est adaptée pour émettre uniquement le faisceau FI1 présentant la longueur d'onde de résonance $\lambda_r$. Aussi, dans le mode de réalisation secondaire le capteur Det effectue l'acquisition d'une seule image : l'image I1. L'unité de traitement UT est alors configurée pour détecter une éventuelle présence du composé à détecter C directement à partir de la première image I1, par une comparaison d'une intensité entre au moins une région Rs et au moins une région Rr. Alternativement, comme dans le mode de réalisation principal, dans le mode de réalisation secondaire cette détection peut être mise en œuvre par une comparaison d'une intensité entre une moyenne des régions Rs et une moyenne des régions Rr.

Comparativement, au mode de réalisation principal, le mode de réalisation secondaire présente l'avantage d'être plus facile à mettre en œuvre car on interroge l'élément interférométrique EI en utilisant un seul faisceau FI1. Il est donc possible d'utiliser une source laser monochromatique par exemple. En effet, en théorie, il est possible d'effectuer la détection du composé C en analysant uniquement l'image I1 dans de très bonnes conditions d'observation. Cela suppose que la différence de réflectivité entre cavités sensibles et les cavités références à la résonance est suffisante pour localiser les régions Rs et Rr dans l'image I1 ou que leur position dans l'image I1 est connue. Cependant, en pratique, le rapport signal sur bruit dans l'image I1 sera faible compte tenu des variations spatiales non contrôlées et/ou aléatoire de la scène imagée par le détecteur. Ainsi, la détection du composé C est plus difficile dans le mode de réalisation secondaire et le taux de faux négatif de détection du composé C sera plus élevé que dans le mode de réalisation principal.

En outre, dans le mode de réalisation principal, l'image I2 est avantageuse car elle facilite grandement l'identification des régions Rs et Rr dans l'image I1.

### Exemple de mise en œuvre de l'élément interférométrique :

Selon un mode de réalisation de l'invention, noté MR1, l'élément interférométrique EI comprend des cavités FP optimisées afin de présenter une résonance à la longueur d'onde de résonance $\lambda_r$ = *612 GHz* qui est la longueur d'onde centrale d'une raie d'absorption du gaz $H_2S$.

Dans ce mode de réalisation, les couches réfléchissantes CR sont réalisées par une couche métallique. Les couches partiellement transparentes CT comprennent chacune un empilement constitué d'une couche inférieure de silicium de 5 $\mu$m d'épaisseur, d'une couche intermédiaire comprenant d'une matrice de pavés métalliques de 47x47 $\mu$m avec un pas de 50 $\mu$m, et d'une couche supérieure d'oxyde de silicium de 2 $\mu$m d'épaisseur. Les couches CT et CR sont séparées d'une distance $L$ = 250 $\mu$m. Cette valeur distance $L$ est proche de $\lambda_r/2$ (245 $\mu$m) mais non strictement égale en raison de la phase de transmission des couches de l'empilement de la couche CT.

De préférence, les éléments métalliques sont réalisés en un métal bon conducteur, tel que le cuivre, l'or ou à défaut l'aluminium, afin de minimiser les pertes associées.

La figure 4 est une représentation graphique de l'évolution du coefficient de réflexion d'une cavité selon le mode de réalisation MR1 en fonction de la longueur d'onde, pour cinq valeurs différentes de concentration en $H_2S$ dans la cavité. Plus précisément, la courbe C7 est obtenue pour une tangente de pertes diélectriques ($\tan\delta$) de 0.01, la courbe C8 est obtenue pour une tangente de pertes diélectriques ($\tan\delta$) de 0.05, la courbe C9 est obtenue pour une tangente de pertes diélectriques ($\tan\delta$) de 0.001, la courbe C10 est obtenue pour une tangente de pertes diélectriques ($\tan\delta$) de 0.0001 et la courbe C11 est obtenue pour une tangente de pertes diélectriques ($\tan\delta$) nulle.

Pour rappel, la tangente de pertes diélectriques est directement reliée à l'absorbance $\alpha$ du composé C par la relation suivante : $\alpha = \frac{\pi}{\lambda}\sqrt{\varepsilon'}\tan\delta$ , où $\varepsilon'$ est la permittivité du composé C. Ainsi, la tangente de perte est proportionnelle à la concentration en espèce C.

Sur la figure 4, on observe que le coefficient de réflexion présente un minimum à $\lambda_r$ = 612 GHz, de profondeur croissante avec la tangente de pertes, soit croissante avec la concentration en gaz. Le coefficient de réflexion hors résonance, en-deçà de 590 GHz ou au-delà de 630 GHz, est proche de 100% pour des concentrations de gaz faibles ou modérées. Sur la courbe C7, on observe que le coefficient de réflexion à la résonance est nul. Il sera donc potentiellement aisé de repérer les régions Rs dans l'image I1 pour cette concentration.

Par des simulations calculant l'évolution des coefficients de réflexion hors et à la résonance en fonction de la concentration en gaz, on détermine les valeurs de réflexion à la résonance et hors résonance pour une concentration de gaz nulle et de 1% présentées dans le tableau suivant (ci-après « tableau 1 »):

| $\tan\delta$ | 0 | 7.2e-5 |
|---|---|---|
| Concentration (%) | 0 | 1% |
| Refl. 580 GHz (%) | 99.35% | 99.32% |
| Refl. à la résonance (%) | 73.29% | 71.51% |

A partir de ces valeurs de réflexion, on peut simuler les images obtenues par une matrice de détecteurs ayant un niveau de bruit de 250 µVrms (valeur typique) et un rapport signal sur bruit (SNR) de 10. A titre d'exemple non limitatif, l'élément EI du mode de réalisation MR1 comprend une matrice de 5x5 cavités FR avec parmi elles 13 cavités références et 12 cavités sensibles disposées en damier.

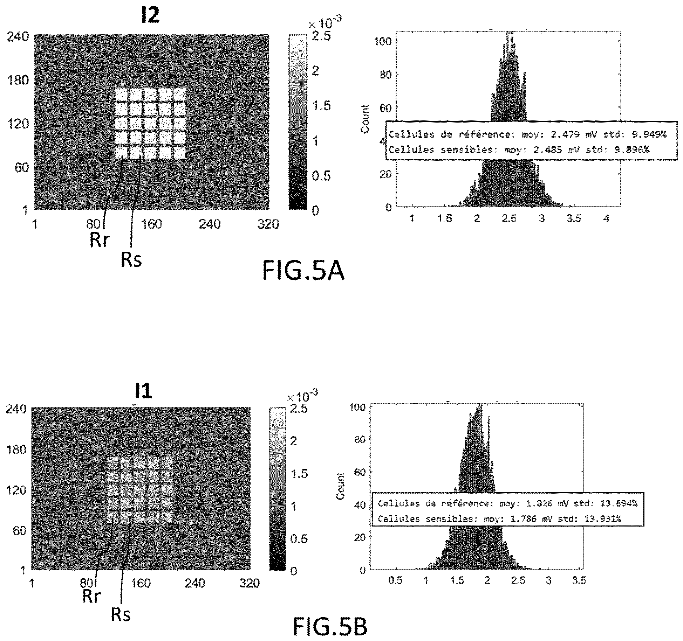

Les figures 5A et 5B présentent les résultats de cette simulation. Plus précisément, la figure 5A présente l'image I2 à gauche et présente l'histogramme des valeurs des pixels des régions Rs et Rr dans l'image I2. La figure 5B présente l'image I1 à gauche et présente l'histogramme des valeurs des pixels des régions Rs et Rr dans l'image I1. A titre d'exemple, les régions Rs et Rr sont chacune sont constitués d'environ 3000 pixels.

On observe tout d'abord que dans les images I1 et 2, il n'est pas possible distinguer les deux types de cavités à l'œil nu et donc de détecter les régions Rs et Rr. En revanche, on peut tracer leur histogramme et calculer leurs valeurs moyennes.

Dans l'histogramme de la figure 5A, on observe que la valeur moyenne des pixels de la région Rs vaut 2.485 mV et est sensiblement égale à la valeur moyenne des pixels de la région Rr qui vaut 2.479 mV. En effet, hors résonance, le coefficient de réflexions des cavités sensibles et celui des cavités références sont quasi-égaux (voir tableau 1).

Dans l'histogramme de la figure 5B, on observe que la valeur moyenne des pixels de la région Rs vaut 1.786 mV et est sensiblement inférieure à la valeur moyenne des pixels de la région Rr qui vaut 1.826 mV. En effet, à la résonance, le coefficient de réflexions des cavités sensibles est inférieur à celui des cavités références compte tenu de l'absorption du composé C présentant une concentration de 1% (voir tableau 1).

Ici, la détection du composé C peut donc être effectuée à partir de l'image I1 uniquement par comparaison de l'intensité moyenne des régions Rs et Rr ou bien en calculant la troisième image à partir de la différence entre l'image I2 et I1, puis en comparant l'intensité moyenne des régions Rs et Rr dans cette troisième image.

La figure 6 illustre l'évolution de la valeur du coefficient de réflexion en fonction de la tangente de pertes à la résonance, pour trois distances L différentes dans une cavité optique selon le mode de réalisation MR1. Plus précisément, la courbe C12 est obtenue pour $L = 2.\lambda_r$, la courbe C13 est obtenue pour $L = \lambda_r$ et la courbe C12 est obtenue pour $L = \lambda_r/2$.

La figure 6 permet d'observer que le choix d'une distance L relativement plus importante permet d'améliorer la sensibilité de l'élément interférométrique EI car une variation de réflexion significative sera obtenue pour une tangente de perte (i.e. concentration en gaz) plus faible. Cela s'explique par le fait qu'une distance L plus importante permet d'avoir une quantité de gaz plus importante dans la cavité.

Aussi, de manière préférentielle, la distance L de séparation entre la couche partiellement transparente et la couche réfléchissante dans les cavités FP de l'interféromètre de l'invention est telle $L = p \times \lambda_r/2$ avec $p \in \mathbb{N}^* > 2$ , préférentiellement p > 4. Cela permet d'améliorer la sensibilité de l'élément interférométrique EI.

La figure 7 illustre un mode de réalisation dans lequel l'élément interférométrique EI comprend deux sous-ensembles de détection SE et SE' chacun optimisé pour une longueur d'onde de résonance respective et différente de l'autre. Ainsi, l'élément interférométrique EI de la figure 7 permet de détecter deux composés C différents ou permet une détection de deux résonances différentes d'un même gaz.

De manière plus générale, selon un mode de réalisation, l'élément interférométrique EI comprend une pluralité de sous-ensembles de détection, chacun optimisé pour une longueur d'onde de résonance respective et différente de l'autre ou des autres longueurs d'onde de résonance.

De manière préférentielle, les longueurs d'onde de résonance sont distantes les unes des autres de moins de 50%. Ainsi, on peut à s'affranchir d'incertitudes de fabrication.

Alternativement, les longueurs d'onde de résonance sont distantes les unes des autres d'au moins 5%. Ainsi, on peut détecter plusieurs composés C différents ou plusieurs résonances différentes d'un même composé.

Comme illustré dans la figure 7, les cavités optiques FP d'un sous-ensemble SE peuvent comprendre une couche diélectrique DA identique disposée entre les couches CT et CR. Cette couche diélectrique DA est préférentiellement formée à partir d'un matériau à faibles pertes afin de réduire l'épaisseur entre les deux surfaces par l'augmentation de l'indice de réfraction moyen du milieu situé entre les couches CT et CR.

De manière encore préférée, l'indice de réfraction et l'épaisseur de la couche diélectrique DA sont adaptés de manière à ce que les cavités optiques présentent une même épaisseur (c'est-à-dire une même dimension selon la direction d'empilement). Cela permet de faciliter la fabrication de l'élément interférométrique.

La figure 8 illustre un objet de l'invention qui est un emballage EA, par exemple alimentaire comprenant un aliment AL. Cet emballage EA comprend l'élément interférométrique EI de l'invention au sein de l'emballage dans le volume où est conservé et disposé l'aliment AL. A titre d'exemple non limitatif, la structure de chacune des cavités FP de l'élément interférométrique EI est optimisée pour présenter une résonance à une absorption résonante du gaz $H_2S$, comme 612 GHz car la détection de sulfure d'hydrogène dans les produits alimentaires périssables tels que viandes ou poissons frais est un indicateur de la dégradation du produit. Ainsi, lorsque l'élément interférométrique EI sera interrogé par une source de lumière SL émettant au moins à la longueur d'onde $\lambda_r$ = 612 *GHz,* il sera possible de détecter une éventuelle altération de l'aliment AL par une présence du gaz $H_2S$.

Il est préférable que la source de lumière SL soit adaptée en fonction de l'emballage alimentaire afin de que ce dernier présente une transmission suffisante (par exemple supérieure à 50%) à la longueur d'onde $\lambda_r$. Par exemple, en gamme térahertz, il sera possible de traverser une paroi en plastique, en papier, en carton, ou encore en tissu.

## Revendications

1. Elément interférométrique (EI) destiné à un dispositif de détection d'au moins un composé (C) présentant une absorption résonante sur une région spectrale prédéterminée centrée sur une longueur d'onde de résonance $\lambda_r$, ledit élément interférométrique comprenant au moins un sous-ensemble de détection (SE) optimisé pour ladite longueur d'onde de résonance $\lambda_r$ et comprenant :

   - au moins deux cavités optiques de type Fabry-Pérot (FP) présentant une résonance à ladite longueur d'onde de résonance $\lambda_r$, chaque cavité comprenant une couche réfléchissante (CR) à ladite longueur d'onde de résonance $\lambda_r$ et une couche partiellement transparente (CT) à ladite longueur d'onde de résonance $\lambda_r$,
   - une couche d'encapsulation (CE) imperméable au(x) composé(s) à détecter (C) et encapsulant au moins une cavité optique, dite cavité référence (REF), de manière à ce que ladite cavité référence soit dépourvue dudit composé à détecter entre la couche réfléchissante (CR) et la couche partiellement transparente, la couche d'encapsulation n'encapsulant pas la seconde cavité optique, dite cavité sensible (SNS), et la couche partiellement transparente de la cavités sensible étant perméable au(x) composé(s) à détecter (C) de manière à ce que la cavité sensible puisse comprendre le ou les composé(s) à détecter (C) entre la couche réfléchissante (CR) et la couche partiellement transparente.

2. Elément interférométrique selon la revendication 1, comprenant une pluralité de cavités sensibles et une pluralité de cavités références, dans lequel les cavités sensibles et les cavités références sont agencées selon une disposition prédéterminée de manière à pouvoir déterminer une position des cavités sensibles et des cavités références par traitement d'une image dudit élément interférométrique.

3. Elément interférométrique selon la revendication précédente, dans lequel ladite disposition prédéterminée est telle que les cavités sensibles et les cavités références sont disposées de manière alternées selon une ligne ou une pluralité de lignes préférentiellement parallèles.

4. Elément interférométrique selon l'une quelconque des revendications précédentes, comprenant une mire optique adaptée de manière à pouvoir déterminer une orientation et une position dudit élément interférométrique par traitement d'une image dudit élément interférométrique.

5. Elément interférométrique selon l'une quelconque des revendications précédentes, dans lequel la ou les cavités sensibles sont adaptées pour présenter un coefficient de réflexion $R_s(\lambda_r)$ à la longueur d'onde de résonance $\lambda_r$ et la ou les cavités références sont adaptées pour présenter un coefficient de réflexion $R_r(\lambda_r)$ à la longueur d'onde de résonance $\lambda_r$ tels que $R_r(\lambda_r) - R_s(\lambda_r) > 1\%$, et préférentiellement $R_r(\lambda_r) - R_s(\lambda_r) > 2\%$, pour une concentration de 1% du ou des composé(s) à détecter (C) entre la couche réfléchissante (CR) et la couche partiellement transparente des cavités sensibles.

6. Elément interférométrique selon l'une quelconque des revendications précédentes, comprenant une pluralité de sous ensemble de détections (SE, SE') chacun optimisé pour une longueur d'onde de résonance respective et différente de l'autre ou des autres longueurs d'onde de résonance.

7. Elément interférométrique selon la revendication précédente, dans lequel les longueurs d'onde de résonance sont distantes les unes des autres de moins de 50%.

8. Elément interférométrique selon la revendication 6, dans lequel les longueurs d'onde de résonance sont distantes les unes des autres d'au moins 5%.

9. Elément interférométrique selon l'une quelconque des revendications 6 à 8, dans lequel les cavités optiques de chaque sous-ensemble de détection comprennent, entre ladite couche partiellement transparente et ladite couche réfléchissante, une couche diélectrique (DA) identique respectivement associée audit sous ensemble de détection, un indice de réfraction et une épaisseur de ladite couche diélectrique respectivement associée audit sous ensemble de détection étant différente d'un indice de réfraction et d'une épaisseur de la ou des couches diélectriques respectivement associée(s) au(x) autre(s) sous ensemble(s) de détection et étant adaptés de manière à ce que les cavités optiques présentent une même épaisseur.

10. Elément interférométrique selon l'une quelconque des revendications précédentes, dans lequel la couche partiellement transparente est séparée d'une distance $p \times \lambda_r/2$, de la couche réfléchissante avec $p \in \mathbb{N}^* > 2$, préférentiellement p > 4.

11. Utilisation dudit élément interférométrique selon l'une quelconque des revendications précédentes, disposé dans un emballage comprenant un aliment, pour détecter une altération dudit aliment.

12. Dispositif (1) de détection d'au moins un composé (C) présentant une absorption résonante sur une région spectrale centrée sur une longueur d'onde de résonance $\lambda_r$, ledit dispositif comprenant :

- une source de lumière (SL) adaptée pour générer un premier faisceau incident (FI1) présentant au moins ladite longueur d'onde de résonance $\lambda_r$
- un élément interférométrique (EI) selon l'une quelconque des revendications 1 à 11 agencé de manière à ce que le premier faisceau illumine la couche partiellement transparente de l'au moins une cavité sensible et la couche partiellement transparente de l'au moins une cavité de référence
- un capteur (Det) comprenant une pluralité de pixels et adapté pour acquérir une image du premier faisceau incident réfléchi par l'élément interférométrique, dite première image,
- une unité de traitement (UT) reliée au capteur et configurée pour détecter une éventuelle présence du ou des composé(s) à détecter (C) à partir d'une comparaison d'une intensité entre, d'une part, au moins une première région de pixels où est détecté le premier faisceau incident réfléchi par ladite au moins une cavité sensible et d'autre part au moins une deuxième région de pixels où est détecté le premier faisceau incident réfléchi par ladite au moins une cavité de référence.

13. Dispositif de détection d'au moins un composé (C) présentant une absorption résonante sur une région spectrale centrée sur une longueur d'onde de résonance $\lambda_r$, ledit dispositif comprenant

- une source de lumière (SL) adaptée pour générer un premier faisceau incident (FI1) présentant au moins ladite longueur d'onde de résonance $\lambda_r$ et pour générer un deuxième faisceau incident (FI2) ne présentant aucune longueur d'onde comprise dans ladite absorption résonante et présentant au moins une longueur d'onde dite longueur d'onde hors résonance $\lambda_{nr}$
- un élément interférométrique (EI) selon l'une quelconque des revendications 1 à 11 agencé de manière à ce que le premier et le deuxième faisceau illuminent la couche partiellement transparente d'au moins une cavité sensible et la couche transparente d'au moins une cavité de référence
- un capteur (Det) comprenant une pluralité de pixels et adapté pour acquérir une image du premier faisceau incident réfléchi par l'élément interférométrique (FR1), dite première image (I1), et une image du deuxième faisceau incident réfléchi par l'élément interférométrique (FR2), dite deuxième image (I2)

- une unité de traitement (UT) reliée au capteur et configurée pour détecter une éventuelle présence du ou des composé(s) à détecter (C) à partir d'une comparaison d'une intensité de la première image et de la deuxième image.

14. Méthode de détection d'au moins un composé (C) présentant une absorption résonante sur une région spectrale centrée sur une longueur d'onde de résonance $\lambda_r$, ladite méthode comprenant les étapes suivantes :

> - générer un premier faisceau incident (FI1) présentant au moins ladite longueur d'onde de résonance $\lambda_r$ et un deuxième faisceau incident (FI2) ne présentant aucune longueur d'onde comprise dans ladite absorption résonante et présentant au moins une longueur d'onde dite longueur d'onde hors résonance $\lambda_{nr}$,
> - illuminer un élément interférométrique (EI) selon l'une quelconque des revendications 1 à 11 avec le premier et le deuxième faisceau de manière à ce qu'ils illuminent la couche partiellement transparente d'au moins une cavité sensible et la couche transparente d'au moins une cavité de référence
> - acquérir une image du premier faisceau incident réfléchi par l'élément interférométrique, dite première image, et une image du deuxième faisceau incident réfléchi par l'élément interférométrique, dite deuxième image
> - détecter une éventuelle présence du ou des composé(s) à détecter (C) à partir d'une comparaison d'une intensité de la première image et de la deuxième image.

15. Méthode selon la revendication précédente, dans lequel ladite détection comprend les étapes suivantes :

> A. calculer une troisième image par la différence entre la deuxième image et la première image
> B. dans la troisième image, comparer l'intensité entre, d'une part, au moins une première région de pixels où est détecté le premier faisceau incident réfléchi par ladite au moins une cavité sensible, et d'autre part, au moins une deuxième région de pixels où est détecté le premier faisceau incident réfléchi par ladite au moins une cavité de référence.

16. Méthode selon la revendication précédente, dans lequel ladite détection comprend une étape A0, mise en œuvre avant ladite étape A, consistant à recaler la première image et la deuxième image.

17. Méthode selon la revendication 15 ou 16, dans lequel ladite détection est effectuée lorsqu'une moyenne $Imoy_1$ de l'intensité de ladite au moins une première région et une moyenne $Imoy_2$ de l'intensité de ladite au moins une deuxième région sont telles que : $\frac{|Imoy_1 - Imoy_2|}{Imoy_1} > S$, avec $S$ compris entre 0.5% et 5%.

18. Méthode selon l'une quelconque des revendications 15 à 17, dans lequel la première image et la deuxième image sont acquises simultanément ou dans un intervalle de temps inférieur à 5 secondes, préférentiellement inférieur à 1 seconde.

**Patentansprüche**

1. Interferometrisches Element (EI), das für eine Vorrichtung zum Detektieren mindestens einer Verbindung (C) bestimmt ist, die eine Resonanzabsorption in einem auf einer Resonanzwellenlänge $\lambda_r$ zentrierten vorbestimmten Spektralbereich aufweist, wobei das interferometrische Element mindestens eine optimierte Detektionsuntereinheit (SE) für die Resonanzwellenlänge $\lambda_r$ umfasst, und umfassend:

> - mindestens zwei optische Hohlräume vom Typ Fabry-Pérot (FP), die eine Resonanz mit der Resonanzwellenlänge $\lambda_r$ aufweist, wobei jeder Hohlraum eine reflektierende Schicht (CR) mit der Resonanzwellenlänge $\lambda_r$ und eine teilweise transparente (CT) Schicht mit der Resonanzwellenlänge $\lambda_r$ umfasst,
> - eine für zu detektierende Verbindung(en) (C) undurchlässige und mindestens einen optischen Hohlraum, Referenzhohlraum (REF) genannt, verkapselnde Verkapselungsschicht (CE), sodass der Referenzhohlraum nicht mit der zu detektierenden Verbindung zwischen der reflektierenden Schicht (CR) und der teilweise transparenten Schicht versehen ist, wobei die Verkapselungsschicht den zweiten optischen Hohlraum, sensibler Hohlraum (SNS) genannt nicht verkapselt, und die teilweise transparente Schicht des sensiblen Hohlraums durchlässig für (die) zu detektierende(n) Verbindung(en) (C) ist, sodass der sensible Hohlraum die zu detektierende(n) Verbindung(en) (C) zwischen der reflektierenden Schicht (CR) und der teilweise transparenten Schicht umfassen kann.

2. Interferometrisches Element nach Anspruch 1, umfassend eine Vielzahl von sensiblen Hohlräumen und eine Vielzahl von Referenzhohlräumen, wobei die sensiblen Hohlräume und die Referenzhohlräume gemäß einer vorbestimmten Anordnung angeordnet sind, um eine Position der sensiblen Hohlräume und der Referenzhohlräume durch Behandeln eines Bildes des interferometrischen Elements bestimmen zu können.

3. Interferometrisches Element nach dem vorstehenden Anspruch, wobei die vorbestimmte Anordnung derart ist, dass die sensiblen Hohlräume und die Referenzhohlräume abwechselnd gemäß einer Linie oder einer Vielzahl von vorzugsweise parallelen Linien angeordnet sind.

4. Interferometrisches Element nach einem der vorstehenden Ansprüche, umfassend ein optisches Visier, das angeordnet ist, um eine Ausrichtung und eine Position des interferometrischen Elements durch Behandeln eines Bildes des interferometrischen Elements bestimmen zu können.

5. Interferometrisches Element nach einem der vorstehenden Ansprüche, wobei der oder die sensible(n) Hohlraum (-räume) angepasst sind, um einen Reflexionskoeffizienten $R_s(\lambda_r)$ mit der Resonanzwellenlänge $\lambda_r$ aufzuweisen, und der oder die Referenzhohlraum (-räume) angepasst sind, um eine Reflexionskoeffizienten $R_r(\lambda_r)$ mit der Resonanzwellenlänge $\lambda_r$ wie $R_r(\lambda_r)$ - $R_s(\lambda_r)$ > 1%, und vorzugsweise $R_r(\lambda_r)$ - $R_s(\lambda_r)$ > 2% bei einer Konzentration von 1% der zu detektierenden Verbindung(en) (C) zwischen der reflektierenden Schicht (CR) und der teilweise transparenten Schicht der sensiblen Hohlräume aufzuweisen.

6. Interferometrisches Element nach einem der vorstehenden Ansprüche, umfassend eine Vielzahl von Detektionsuntereinheiten (SE, SE'), die jeweils für eine jeweilige Resonanzwellenlänge optimiert sind und sich von der anderen oder den anderen Resonanzwellenlängen unterscheiden.

7. Interferometrisches Element nach dem vorstehenden Anspruch, wobei die Resonanzwellenlängen um mindestens 50% voneinander beabstandet sind.

8. Interferometrisches Element nach Anspruch 6, wobei die Resonanzwellenlängen um mindestens 5% voneinander beabstandet sind.

9. Interferometrisches Element nach einem der Ansprüche 6 bis 8, wobei die optischen Hohlräume einer jeden Detektionsunterheit zwischen der teilweise transparenten Schicht und der reflektierenden Schicht eine identische dielektrische Schicht (DA) umfassen, die jeweils der Detektionsuntereinheit zugeordnet sind, wobei sich ein Brechungsindex und eine Dicke der dielektrischen Schicht, die jeweils der Detektionsuntereinheit zugeordnet ist, von einem Brechungsindex und einer Dicke der dielektrischen Schicht(en) unterscheiden, die jeweils der (den) Detektionsuntereinheit(en) zugeordnet sind, und angepasst sind, sodass die optischen Hohlräume eine gleiche Dicke aufweisen.

10. Interferometrisches Element nach einem der vorstehenden Ansprüche, wobei die teilweise transparente Schicht um eine Distanz p $\times$ $\lambda_r$/2, von der reflektierenden Schicht getrennt ist, mit $p \in \mathbb{N}^* > 2$, vorzugsweise p > 4.

11. Verwendung des interferometrischen Elements nach einem der vorstehenden Ansprüche, das in einer Verpackung angeordnet ist, die ein Nahrungsmittel umfasst, um eine Veränderung des Nahrungsmittels zu detektieren.

12. Vorrichtung (1) zum Detektieren mindestens einer Verbindung (C), die eine Resonanzabsorption in einem auf einer Resonanzwellenlänge $\lambda_r$ zentrierten vorbestimmten Spektralbereich aufweist, wobei die Vorrichtung umfasst:

- eine Lichtquelle (SL) die angepasst ist, um ein erstes einfallendes Strahlenbündel (FI1) zu erzeugen, das mindestens die Resonanzwellenlänge $\lambda_r$ aufweist
- ein interferometrisches Element (EI) nach einem der Ansprüche 1 bis 11, das angeordnet ist, sodass das erste Strahlenbündel die teilweise transparente Schicht des mindestens einen sensiblen Hohlraumes und die teilweise transparente Schicht des mindestens einen Hohlraumes erleuchtet
- einen Messfühler (Det), der eine Vielzahl von Pixeln umfasst und angepasst ist, um ein Bild des ersten einfallenden, von dem interferometrischen Element reflektierten Strahlenbündel, erstes Bild genannt, aufzunehmen,
- eine Behandlungseinheit (UT), die an den Messfühler angeschlossen ist und konfiguriert ist, um ein eventuelles Vorhandensein der zu detektierenden Verbindung(en) (C) aus einem Vergleich einer Intensität zwischen, einerseits, mindestens einem ersten Pixelbereich, in dem das erste einfallende, von dem mindestens einen sensiblen Hohlraum reflektierte Strahlenbündel detektiert wird, und andererseits mindestens einem zweiten Pixelbereich, in dem das erste einfallende, von dem mindestens einen Referenzhohlraum reflektierte Strahlenbündel detektiert wird, zu detektieren.

13. Vorrichtung zum Detektieren mindestens einer Verbindung (C), die eine Resonanzabsorption in einem auf einer Resonanzwellenlänge $\lambda_r$ zentrierten vorbestimmten Spektralbereich aufweist, wobei die Vorrichtung umfasst

- eine Lichtquelle (SL), die angepasst ist, um ein erstes einfallendes Strahlenbündel (FI1) zu erzeugen, das mindestens die Resonanzwellenlänge $\lambda_r$ aufweist, und um ein zweites einfallendes Strahlenbündel (FI2) zu erzeugen, das keine Wellenlänge aufweist, die in der Resonanzabsorption umfasst ist, und mindestens eine Außer-Resonanzwellenlänge $\lambda_{nr}$ genannte Wellenlänge aufweist,
- ein interferometrisches Element (EI) nach einem der Ansprüche 1 bis 11, das angeordnet ist, sodass das erste und das zweite Strahlenbündel die teilweise transparente Schicht mindestens eines sensiblen Hohlraumes und die transparente Schicht mindestens eines Hohlraumes erleuchten
- einen Messfühler (Det), der eine Vielzahl von Pixeln umfasst und angepasst ist, um ein Bild des ersten einfallenden, von dem interferometrischen Element (FR1) reflektierten Strahlenbündel, erstes Bild (11) genannt, und ein Bild des zweiten einfallenden, von dem interferometrischen Element (FR2) reflektierten Strahlenbündel, zweites Bild (12) genannt, aufzunehmen
- eine Behandlungseinheit (UT), die an der Messfühler angeschlossen ist und konfiguriert ist, um ein eventuelles Vorhandensein der zu detektierenden Verbindung(en) (C) aus einem Vergleich einer Intensität des ersten Bildes und des zweiten Bildes zu detektieren.

14. Verfahren zum Detektieren mindestens einer Verbindung (C), die eine Resonanzabsorption in einem auf einer Resonanzwellenlänge $\lambda_r$ zentrierten vorbestimmten Spektralbereich aufweist, wobei das Verfahren die folgenden Schritte umfasst:

- Erzeugen eines ersten einfallenden Strahlenbündels (FI1), das mindestens die Resonanzwellenlänge $\lambda_r$ aufweist, und eines zweiten einfallenden Strahlenbündels (FI2), das keine Wellenlänge aufweist, die in der Resonanzabsorption umfasst ist, und mindestens eine Außer-Resonanzwellenlänge $\lambda_{nr}$ genannte Wellenlänge aufweist,
- Erleuchten eines interferometrischen Elements (EI) nach einem der Ansprüche 1 bis 11 mit dem ersten und dem zweiten Strahlenbündel, sodass sie die teilweise transparente Schicht mindestens eines sensiblen Hohlraumes und die transparente Schicht mindestens eines Hohlraumes erleuchten

- Aufnehmen eines Bildes des ersten einfallenden, von dem interferometrischen Element reflektierten Strahlenbündel, erstes Bild genannt, und eines Bildes des zweiten einfallenden, von dem interferometrischen Element reflektierten Strahlenbündel, zweites Bild genannt
- Detektieren eines eventuellen Vorhandenseins der zu detektierenden Verbindung(en) (C) aus einem Vergleich einer Intensität des ersten Bildes und des zweiten Bildes.

15. Verfahren nach dem vorstehenden Anspruch, wobei die Detektion die folgenden Schritte umfasst:

A. Berechnen eines dritten Bildes durch die Differenz zwischen dem zweiten Bild und dem ersten Bild
B. Vergleichen im dritten Bild einer Intensität zwischen, einerseits, mindestens einem ersten Pixelbereich, in dem das erste einfallende, von dem mindestens einen sensiblen Hohlraum reflektierte Strahlenbündel detektiert wird und andererseits mindestens einem zweiten Pixelbereich, in dem das erste einfallende, von dem mindestens einen Referenzhohlraum reflektierte Strahlenbündel detektiert wird.

16. Verfahren nach dem vorstehenden Anspruch, wobei die Detektion einen Schritt A0 umfasst, der vor dem Schritt A umgesetzt wird, der darin besteht, das erste Bild und das zweite Bild nachzujustieren.

17. Verfahren nach Anspruch 15 oder 16, wobei die Detektion durchgeführt wird, wenn ein Mittelwert $Imoy_1$ der Intensität des mindestens einen ersten Bereichs und ein Mittelwert $Imoy_2$ der Intensität des mindestens einen zweiten Bereichs derart sind,

dass: $\dfrac{|Imoy_1 - Imoy_2|}{Imoy_1} > S$ wobei $S$ zwischen 0,5% und 5% umfasst ist.

18. Verfahren nach einem der Ansprüche 15 bis 17, wobei das erste Bild und das zweite Bild gleichzeitig oder in einem Zeitintervall von weniger als 5 Sekunden, vorzugsweise weniger als 1 Sekunde aufgenommen werden.

## Claims

1. Interferometer element (EI) intended for a device for the detection of at least one compound (C) having a resonant absorption over a predetermined spectral region centered on a resonance wavelength $\lambda_r$, said interferometer element comprising at least one detection sub-assembly (SE) optimized for said resonance wavelength $\lambda_r$ and comprising:

- at least two Fabry-Pérot optical cavities (FP) having a resonance at said resonance wavelength $\lambda_r$, each cavity comprising a reflective layer (CR) at said resonance wavelength $\lambda_r$ and a partially transparent layer (CT) at said resonance wavelength $\lambda_r$,
- an encapsulation layer (CE) impermeable to the compound(s) to be detected (C) and encapsulating at least one optical cavity, referred to as the reference cavity (REF), such that said reference cavity is free of said compound to be detected between the reflective layer (CR) and the partially transparent layer, the encapsulation layer not encapsulating the second optical cavity, referred to as the sensitive cavity (SNS), and the partially transparent layer of the sensitive cavities being permeable to the compound(s) to be detected (C) such that the sensitive cavity may comprise the compound(s) to be detected (C) between the reflective layer (CR) and the partially transparent layer.

2. Interferometer element according to claim 1, comprising a plurality of sensitive cavities and a plurality of reference cavities, wherein the sensitive cavities and the reference cavities are arranged according to a predetermined layout as to be able to determine a position of the sensitive cavities and the reference cavities by processing an image from said interferometer element.

3. Interferometer element according to the preceding claim, wherein said predetermined layout is such that the sensitive cavities and the reference cavities are arranged alternately according to a line or a plurality of preferably parallel lines.

4. Interferometer element according to any one of the preceding claims, comprising an optical test pattern adapted as to be able to determine an orientation and a position of said interferometer element by processing an image from said interferometer element.

5. Interferometer element according to any one of the preceding claims, wherein the sensitive cavity or cavities are adapted to have a reflection coefficient $R_s(\lambda_r)$ at the resonance wavelength $\lambda_r$ and the reference cavity or cavities are adapted to have a reflection coefficient $R_r(\lambda_r)$ at the resonance wavelength $\lambda_r$ such as $R_r(\lambda_r) - R_s(\lambda_r) > 1\%$, and preferably $R_r(\lambda_r) - R_s(\lambda_r) > 2\%$, for a concentration of 1% of the compound(s) to be detected (C) between the reflective layer (CR) and the partially transparent layer of the sensitive cavities.

6. Interferometer element according to any one of the preceding claims, comprising a plurality of detection sub-assemblies (SE, SE') each optimized for a respective resonance wavelength different from one another or other resonance wavelengths.

7. Interferometer element according to the preceding claim, wherein the resonance wavelengths are spaced no more than 50% apart from one another.

8. Interferometer element according to claim 6, wherein the resonance wavelengths are spaced no more than 5% apart from one another.

9. Interferometer element according to any one of claims 6 to 8, wherein the optical cavities of each detection sub-assembly comprise, between said partially transparent layer and said reflective layer, an identical dielectric layer (DA) respectively associated with said detection sub-assembly, one refractive index and one thickness of said dialectric layer respectively associated with said detection sub-assembly being different from one refractive index and one thickness of the dielectric layer(s) respectively associated with the other detection sub-assembly(ies) and adapted so that the optical cavities have the same thickness.

10. Interferometer element according to any one of the preceding claims, wherein the partially transparent layer is separated by a distance p $\times$ $\lambda_r/2$, from the reflective layer with $p \in \mathbb{N}^* > 2$, preferably p > 4.

11. Use of said interferometer element according to any one of the preceding claims, placed in a packaging comprising a food product, in order to detect an alteration in said food product.

12. Device for detecting (1) at least one compound (C) having a resonant absorption over a spectral region centered on a resonance wavelength $\lambda_r$, said device comprising:

- a light source (SL) adapted to generate a first incident beam (FI1) having at least said resonance wavelength $\lambda_r$
- an interferometer element (EI) according to any one of claims 1 to 11 arranged so that the first beam illuminates the partially transparent layer of at least one sensitive cavity and the partially transparent layer of at least one reference cavity
- a sensor (Det) comprising a plurality of pixels and adapted to acquire an image from the first incident beam reflected by the interferometer element, referred to as the first image,
- a processing unit (UT) connected to the sensor and configured to detect any presence of the compound(s) to be detected (C) based on a comparison of an intensity between, on the one hand, at least one first pixel region where the first incident beam is detected reflected by said at least one sensitive cavity and on the other hand, at least a second pixel region where the first incident beam is detected reflected by said at least one reference cavity.

13. Device for detecting at least one compound (C) having a resonant absorption over a spectral region centered on a resonance wavelength $\lambda_r$, said device comprising

- a light source (SL) adapted to generate a first incident beam (FI1) having at least said resonance wavelength $\lambda_r$ and to generate a second incident beam (FI2) having no wavelength comprised in said resonant absorption and having at least one wavelength referred to as the off-resonance wavelength $\lambda_{nr}$,
- an interferometer element (EI) according to any one of claims 1 to 11 arranged so that the first and the second beam illuminate the partially transparent layer of at least one sensitive cavity and the partially transparent layer of at least one reference cavity
- a sensor (Det) comprising a plurality of pixels and adapted to acquire an image from the first incident beam reflected by the interferometer element (FR1), referred to as the first image (I1), and an image of the second incident beam reflected by the interferometer element (FR2), referred to as the second image (I2)
- a processing unit (UT) connected to the sensor and configured to detect any presence of the compound(s) to be detected (C) based on a comparison of an intensity of the first image and the second image.

14. Method for detecting at least one compound (C) having a resonant absorption over a spectral region centered on a resonance wavelength $\lambda_r$, said method comprising the following steps:

- generating a first incident beam (FI1) having at least said resonance wavelength $\lambda_r$ and a second incident beam (FI2) having no wavelength comprised in said resonant absorption and having at least one wavelength referred to as the off-resonance wavelength $\lambda_{nr}$,
- illuminating an interferometer element (EI) according to any one of claims 1 to 11 with the first and the second beam in a manner that they illuminate the partially transparent layer of at least one sensitive cavity and the transparent layer of at least one reference cavity
- acquiring an image of the first incident beam reflected by the interferometer element, referred to as the first image, and a second incident beam reflected by the interferometer element, referred to as the second image
- detecting any presence of the compound(s) to be detected (C) based on a comparison of an intensity of the first image and the second image

15. Method according to the preceding claim, wherein said detection comprises the following steps:

A. calculating a third image by the difference between the second image and the first image
B. in the third image, comparing the intensity between, on the one hand, at least one first pixel region where the first incident beam is detected reflected by said at least one sensitive cavity, and on the other hand, at least one second pixel region where the first incident beam is detected reflected by said at last one reference cavity.

16. Method according to the preceding claim, wherein said detection comprises step A0, implemented before said step A, consisting of readjusting the first image and the second image.

17. Method according to claims 15 or 16, wherein said detection is carried out when an average $Imoy_1$ of the intensity of said at least one first region and an average $Imoy_2$ of the intensity of said at least one second region are such that: $\frac{|Imoy_1 - Imoy_2|}{Imoy_1} > S$

with $S$ is between 0.5% and 5%.

18. Method according to any one of claims 15 to 17, wherein the first image and the second image are acquired simultaneously or within a time interval of below 5 seconds, preferably below 1 second.

FIG.1

FIG.2A

FIG.2B

FIG.2C

FIG.3A

FIG.3B

FIG.4

FIG.5A

FIG.5B

FIG.6

FIG.7

FIG.8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2021018431 A

**Littérature non-brevet citée dans la description**

- **VOIR L. KUULIALA et al.** Spoilage evaluation of raw Atlantic salmon (Salmo salar) stored under modified atmospheres by multivariate statistics and augmented ordinal regression. *International Journal of Food Microbiology*, August 2019, vol. 303, 46-57